# FASCICULE DE BREVET EUROPEEN

(11) **EP 1 265 617 B1**
(45) Date de publication et mention de la délivrance du brevet: **21.06.2006**
(21) Numéro de dépôt: 01969895.0
(22) Date de dépôt: 14.09.2001
(51) Int. Cl.: A61K 31/57, A61K 31/565, A61K 9/06, A61K 9/70, A61K 47/00

(54) **NOUVELLES COMPOSITIONS ESTRO-PROGESTATIVES TOPIQUES A EFFET SYSTEMIQUE**
TOPISCH ANZUWENDENDE ÖSTROGEN/PROGESTIN ENTHALTENDE ARZNEIMITTEL MIT SYSTEMISCHER WIRKUNG
NOVEL TOPICAL OESTROPROGESTATIONAL COMPOSITIONS WITH SYSTEMIC EFFECT

(30) Priorité: 15.09.2000 FR 0011791
(43) Date de publication de la demande: 18.12.2002
(73) Titulaire: Laboratoire Theramex SAM, 98000 Monaco (MC)
(72) Inventeur: GRAY, Georges, MC-98000 Monaco (MC); VILLET, Bertrand, 92290 Chatenay-Malabry (FR); PARIS, Jacques, Le Clos de Cimiez, F-06100 Nice (FR); THOMAS, Jean-Louis, F-94220 Charenton-le-Pont (FR)
(74) Mandataire: Burtin, Jean-François
(86) Numéro de dépôt international: PCT/FR2001/002865
(87) Numéro de publication internationale: WO 2002/022132

(56) Documents cités:
- EP-A- 0 785 211
- EP-A- 0 785 212
- WO-A-01/30357
- WO-A-01/30358
- FR-A- 2 737 411
- FR-A- 2 754 179
- FR-A- 2 776 191

## Description

La présente invention se rapporte au domaine de la chimie thérapeutique et plus spécialement à la réalisation de nouvelles formes galéniques destinées à être appliquées sur la peau.

La présente invention a plus particulièrement pour objet des préparations topiques cutanées dont les principes actifs sont un progestatif de synthèse et un estrogène naturel ou synthétique et dont le pouvoir pénétrant permet d'obtenir un effet hormonal systémique.

L'invention concerne plus spécifiquement une composition estroprogestative topique à effet systémique pour le traitement hormonal de la périménopause et de la ménopause ainsi que pour le traitement du déficit hormonal ovarien chez la femme en aménorrhée.
Le brevet français FR 2 737 411 et le brevet français FR 2 754 179 au nom du Demandeur décrivent déjà des compositions estroprogestatives destinées à l'administration par voie orale.
Cependant, aussi bien pour les progestatifs que pour les estrogènes, la voie orale n'est pas sans présenter quelques inconvénients. D'une part, elle oblige à administrer des doses importantes afin de compenser la dégradation des principes actifs lors du passage dans l'intestin et le foie (effet dit « de premier passage »). D'autre part, elle ne conduit pas à des taux plasmatiques constants au cours du temps puisque la prise orale est suivie d'un pic plasmatique au cours duquel les concentrations sanguines sont transitoirement élevées.

L'estradiol et certains de ses dérivés ainsi que certains progestatifs de synthèse sont administrés par voie percutanée pour obtenir soit une substitution dans le cadre d'un traitement hormonal substitutif, soit un effet contraceptif. Cependant, cela demande, en particulier pour les progestatifs, l'utilisation de formes galéniques particulières telles que des patches associant occlusion, agents solubilisants et promoteurs de passage puissants pour obtenir les taux plasmatiques nécessaires à un effet systémique. Ces solutions techniques ne sont pas disponibles pour tous les progestatifs. De plus, les principes actifs interagissent sur le passage percutané respectif. De ce fait, les préparations précédemment décrites pour le passage percutané d'un agent progestatif de synthèse seul, comme décrit dans la demande de brevet français publiée sous le N° 2 776 191 au nom du Demandeur, ne sont pas directement applicables à une association estro-progestative car chaque association de ce type demande une solution galénique particulière.
La fonction de barrière protectrice de la peau contre les agressions extérieures rend celle-ci difficilement perméable vis-à-vis de nombreuses substances et ne laisse pénétrer les molécules médicamenteuses que sous certaines conditions : taille et nature de la molécule, solubilité, stabilité, nature du véhicule contenant la molécule ; épaisseur de la peau, état d'hydratation, état pathologique, localisation..
Ainsi, la libération d'un principe actif à partir d'un véhicule et sa pénétration à travers la peau jusqu'à la circulation sanguine ou lymphatique dépend de nombreux paramètres physico-chimiques et physiologiques qui sont rarement réunis ou qui restent encore à définir.

Dans la présente invention, la nature même de l'un des 2 principes actifs, à savoir le progestatif de synthèse, représente le principal obstacle à la pénétration percutanée ; ainsi, le problème majeur qui se pose pour le progestatif est sa faible diffusion à travers l'épiderme du fait de son caractère lipophile. Une deuxième difficulté réside dans l'interaction entre les deux principes actifs vis à vis du passage percutané. Le choix du véhicule utilisé dans les compositions conformes à l'invention a donc une grande importance sur la pénétration percutanée et l'activité thérapeutique des principes actifs.
Les compositions topiques selon l'invention permettent d'obtenir un effet systémique par optimisation du passage percutané d'un progestatif de synthèse dérivé de la 19-nor progestérone et d'un estrogène naturel ou synthétique, associés dans la même composition.

La présente invention a donc plus spécifiquement pour objet une composition hormonale topique à effet systémique pour le traitement hormonal de la périménopause et de la ménopause ainsi que pour le traitement du déficit hormonal ovarien chez la femme en aménorrhée, sous forme de gel ou de solution, caractérisée en ce qu'elle comprend :
- à titre de principes actifs, un progestatif dérivé de la 19-nor progestérone et un estrogène, naturel ou synthétique
- ainsi qu'un véhicule permettant le passage systémique desdits principes actifs choisi dans le groupe constitué par un agent solubilisant, un agent promoteur d'absorption, un agent filmogène, un agent gélifiant et leurs mélanges,
en association ou en mélange avec des excipients appropriés pour la réalisation d'une forme pharmaceutique gélifiée et/ou filmogène.

Les compositions conformes à l'invention pourront se trouver sous forme de gel, de gel filmogène ou de solution filmogène.
Le progestatif dérivé de la 19-nor progestérone utilisé dans la présente invention est le nomégestrol et/ou un de ses esters ou un de ses éthers. Un exemple d'éther de nomegestrol est l'éther tétrahydropyranique de nomegestrol. Un exemple d'ester de nomegestrol est l'acétate de nomegestrol.

L'estrogène utilisé dans la présente invention est l'estradiol 17beta ou un de ses esters ou éthers. Un exemple d'ester d'acide gras d'estradiol est le valérate d'estradiol. Un exemple d'éther d'estradiol est le promestriène (éther 17-méthylique et 3-propylique d'estradiol).

Administré dans les compositions conformes à l'invention, l'acétate de nomégestrol et l'estradiol sont capables de traverser la peau et de passer dans la circulation sanguine pour donner des taux plasmatiques détectables à l'aide des méthodes utilisées pour le doser dans les milieux biologiques.
Les taux plasmatiques d'estradiol et d'acétate de nomégestrol obtenus avec les compositions qui font l'objet de la présente invention sont à même d'induire un effet hormonal sur les tissus situés à distance du lieu d'application et notamment sur l'endomètre.

L'association d'estradiol et d'acétate de nomégestrol, ainsi administrée de façon réitérée, produit une action thérapeutique lorsqu'elle est appliquée chez des femmes non ménopausées souffrant d'une insuffisance estro-progestative ou chez des femmes ménopausées candidates à une hormonothérapie substitutive.

Selon la présente invention, le nomegestrol ou un de ses esters ou éthers est présent en une quantité variant de 0,05 à 5,0 %, et de préférence de 0,05 à 1,5 % en poids de la composition totale. L'estradiol ou un de ses esters ou éthers est présent en une quantité variant de 0,05 à 1,0 % en poids de la composition totale, et de préférence de 0,05 à 0,5 % en poids.

D'une manière davantage préférée, le nomegestrol ou un de ses esters ou de ses éthers est présent en une quantité variant de 0,1 à 2 % en poids de la composition totale et l'estradiol ou un de ses esters ou éthers en une quantité variant de 0,1 à 0,3 % en poids de la composition totale, avec une préférence pour une concentration en nomegestrol ou un de ses esters ou éthers variant de 0,1 à 1 % et l'estradiol de 0,01 à 0,2 %.

Les compositions topiques à effet systémique actuellement préférées selon l'invention sont celles contenant une quantité de nomegestrol ou d'un de ses esters ou éthers de l'ordre de 1,0 % en poids de la composition totale, et une quantité d'estradiol ou d'un de ses esters ou éthers de l'ordre de 0,15 % en poids de la composition totale.

Les agents solubilisants et les agents promoteurs d'absorption ont des modes d'action différents mais les uns et les autres-permettent de favoriser la pénétration des principes actifs à travers la peau. Les agents solubilisants, par une action sur l'activité thermodynamique de la molécule active, améliorent la solubilité du principe actif et modifient son affinité pour les structures cutanées, en particulier le stratum corneum. Les agents promoteurs d'absorption diminuent la résistance à la diffusion de la barrière cutanée en modifiant sa structure.

### Les agents solubilisants

Les agents solubilisants augmentent la solubilité des principes actifs mais il n'y a cependant pas de relation directe entre l'amélioration de la solubilité du principe actif dans le véhicule et l'augmentation de son passage percutané. En effet, les agents qui améliorent la solubilité d'un principe actif augmentent également son affinité pour le véhicule et peuvent donc en définitive diminuer sa diffusion à travers la peau.
Pour qu'un principe actif soit totalement solubilisé dans un véhicule, il doit avoir une certaine affinité pour celui-ci ; néanmoins, cette affinité ne doit pas être trop importante afin que le coefficient de partage du principe actif soit en faveur de sa diffusion à travers la peau. Selon la présente invention, le choix des solvants et de leurs concentrations assure la dissolution et un passage percutané adéquat pour chacun des deux principes actifs.

Selon la présente invention, des exemples d'agents solubilisants appropriés sont les alcools, les mélanges hydroalccoliques, le propylèneglycol, le polyéthylèneglycol, le polyéthylène 20 sorbitane mono-oléate [commercialisé par exemple sous la dénomination Polysorbate 80 DF], un glycéride en C₈/C₁₀ polyoxyéthyléné glycosylé (commercialisé par exemple sous la dénomination Labrasol®). On emploie généralement; à titre d'agent solubilisant, un mélange de solvants et/ou des agents solubilisants précités, qui, grâce à une synergie d'action, est plus efficace que chacun d'entre eux utilisé seul.

De préférence, l'agent solubilisant est choisi dans le groupe constitué par l'eau, les alcools, le propylèneglycol ou leurs mélanges.
Un exemple d'agent solubilisant approprié pour la composition topique à effet systémique selon l'invention est le mélange ternaire éthanol/ eau/ propylèneglycol, dans lequel la quantité d'éthanol varie de 30 à 60 % en poids de la composition totale, la quantité d'eau varie de 20 à 60 % en poids de la composition totale, et plus particulièrement de 30 à 60 % en poids, et celle de propylèneglycol de 2 à 20 % en poids de la composition totale.
Plus particulièrement, un agent solubilisant approprié pour la composition conforme à l'invention est le mélange comprenant 40 à 60 % d'éthanol, 25 à 45 % d'eau et 6 à 12 % de propylèneglycol, et en particulier de 40 à 50 % d'éthanol, de 40 à 45 % d'eau et de 6 à 12 % en poids de propylèneglycol.

### Les agents promoteurs d'absorption

Les agents promoteurs d'absorption sont des substances capables d'améliorer la diffusion des principes actifs dans l'épiderme, en particulier le stratum corneum. Ces adjuvants peuvent être classés en différentes familles en fonction de leur structure chimique. A titre d'exemple de promoteurs d'absorption, on pourra citer des dérivés de dioxolane tels que l'isopropylidène glycerol commercialisé sous la dénomination Solketal (qui est également un excellent agent solubilisant pour les hormones de cette invention) ou le 2n-nonyl 1-3 dioxolane ; ou bien l'éther monoéthylique du diéthylène glycol (par exemple celui commercialisé sous la dénomination Transcutol®). Des promoteurs d'absorption sont également décrits dans les familles chimiques suivantes : polyols, acides gras, esters d'acides gras, alcools et amides. A titre d'exemple de substances représentatives de ces familles, on peut citer notamment le monocaprylate de propylène glycol ou Capryol 90, l'acide caprylique, l'adipate de diisopropyle, le polysorbate 80, l'octyl-2 dodécanol et la 1-dodécylazacyclohepta-2-one ou Azone. Des substances présentant des propriétés de promoteurs d'absorption peuvent également être trouvées dans la famille des sulfoxydes (comme par exemple le diméthylsulfoxyde), des terpènes (par exemple le d-limonène), des alkanes (par exemple le N-heptane) ou parmi les acides organiques (acide salicylique et salicylates notamment).
L'agent promoteur d'absorption convenant plus particulièrement dans la présente invention est choisi dans le groupe des dioxolanes, comme par exemple l'isopropylidèneglycérol (Solkétal).
La quantité d'agent promoteur d'absorption dans les compositions conformes à l'invention varie de 2 à 12 % en poids de la composition totale.
Un agent promoteur d'absorption convenant particulièrement dans les compositions selon l'invention est l'isopropylidèneglycérol ; la quantité d'isopropylidèneglycérol est de préférence de 3 à 8 % en poids de la composition totale, et d'une manière davantage préférée de 3 à 6 % en poids.

### Les agents gélifiants

Le choix des agents gélifiants et des agents filmogènes est également important dans les compositions selon l'invention.

Les agents gélifiants sont des substances qui épaississent et modifient la viscosité d'un véhicule liquide, constituant ainsi un gel sous la forme d'un réseau colloïdal tridimensionnel. Il existe plusieurs sortes d'agents gélifiants : les agents gélifiants naturels (minéraux, végétaux, animaux), les agents synthétiques et les agents semi-synthétiques.
Des exemples d'agents gélifiants naturels sont la gomme guar, les extraits d'algues (les alginates, les carraghénates, la gélose), les polysaccharides (la gomme xanthane, la gomme arabique, la gomme adragante), les amidons, les pectines, etc.
Des exemples d'agents gélifiants synthétiques ou semi-synthétiques sont les dérivés cellulosiques, notamment ceux obtenus par estérification ou par éthérification de la cellulose, et les dérivés acryliques. Dans la catégorie des dérivés acryliques, on trouve les carbomères, les polycarbophiles, les acrylates.
Selon la présente invention, l'agent gélifiant est choisi de préférence dans le groupe constitué par les dérivés cellulosiques et les dérivés acryliques.

Parmi les dérivés cellulosiques, on trouve les méthylcelluloses (Methocel, Metolose), les éthylcelluloses (Ethocel, Aquacoat®), les hydroxypropylméthylcelluloses (Kenal, Methocel, Hypromelose), les hydroxyéthylcelluloses (Cellosize, Natrosol), les hydroxypropylcelluloses (Klucel), les carboxyméthylcelluloses, réticulées ou non, sous forme sodique ou calcique (Akucell, Nymcel, Tylose CB, Croscarmellose, Acdisol).
Parmi les dérivés acryliques, on citera notamment les carbomères, en particulier ceux commercialisés sous les dénominations Carbopol ® ou Synthalen ®. Selon l'invention les carbomères préférés sont ceux qui forment des gels qui se « cassent » le plus facilement au contact des électrolytes et de la peau.
Les carbomères donnent des formulations stables dans le temps et confèrent à la formulation des propriétés rhéologiques reproductibles du fait de leur nature synthétique.
L'existence de différents degrés ou grades pour les produits tient à la différence de poids moléculaire, au degré de réticulation, à la nature des arrangements moléculaires et au solvant de polymérisation.
Ainsi, parmi les différents grades de carbomère, on pourra citer ceux commercialisés par la société Goodrich sous les dénominations Carbopol 974 P ®, Carbopol 980 ®, Carbopol 1382 ® et Carbopol 2020 ®, ou des produits similaires comme les Synthalen de 3 V France, tels quels (Synthalen K, L, M) ou préneutralisés, comme par exemple les Synthalen PNC ®.

Selon la présente invention, le carbomère commercialisé sous la dénomination Carbopol 1382® est particulièrement approprié car il se fluidifie au contact des électrolytes de la peau et évite ainsi un dépôt de polymère qui risquerait de faire obstacle au passage des principes actifs. La quantité de Carbopol 13 82 est de préférence de 0,3 à 1 % en poids de la composition totale.

### Les agents filmogènes

Les agents filmogènes utilisés sont ceux qui sont employés pour réaliser des solutions d'enrobage ou de pelliculage car ils sont pour la plupart d'entre eux issus de l'industrie alimentaire ou biomédicale. Ils permettent d'envisager une application cutanée en vue de réaliser un film occlusif au niveau de la peau, et de conserver une hydratation cutanée connue pour favoriser le passage percutané. De plus, les agents filmogènes permettent de donner un toucher plus agréable à la formulation.

Les agents filmogènes peuvent être classés en différents groupes en fonction de leur solubilité. Selon la présente invention, l'agent filmogène est choisi dans le groupe constitué par les silicones, les dérivés cellulosiques, les dérivés méthacryliques et les dérivés de la polyvinylpyrrolidone.

Les silicones utilisées conformément à l'invention peuvent être solubles ou insolubles dans l'eau. Selon la présente invention, la silicone est choisie dans le groupe constitué par la diméthicone, le diméthiconol, la siméthicone, leurs mélanges, et plus particulièrement :
- le mélange de stéaroxytriméthylsilane et d'alcool stéarique commercialisé sous le nom de Silky Wax 10 (Dow Corning) en présence ou non d'émulsifiant comme la laurylmethicone,
- le mélange dimethiconol (hydroxy(n-blocked)polydiméthylsiloxane) dans la dimethicone (polydimethyl-siloxane) commercialisé sous la dénomination DC Blend 20.
La quantité de silicone varie de 1 à 3 % en poids de la composition totale.
Dans la présente invention, la silicone particulièrement appropriée est celle commercialisée sous la dénomination DC Blend 20 ; la quantité de DC Blend 20 particulièrement appropriée est de l'ordre de 2 % en poids de la composition totale.

Parmi les dérivés cellulosiques, on pourra citer :
- l'acétate succinate d'hydroxypropylméthylcellulose, et notamment celui commercialisé par la société Seppic sous la dénomination Aqoat AS-LF®,
- une dispersion aqueuse d'acétophtalate de cellulose contenant 70 % d'eau, 23 % d'acétophtalate de cellulose et 7 % de poloxamer, et notamment celle commercialisée par la société Seppic sous la dénomination Aquacoat CPD®,
- une dispersion aqueuse d'éthylcellulose, d'alcool cétylique et de lauryl sulfate de sodium, et notamment celle commercialisée par la société Seppic sous la dénomination Aquacoat ECD 30®,
- l'éthylcellulose.
Parmi les dérivés méthacryliques, on pourra citer :
- une dispersion aqueuse d'un copolymère anionique d'acide méthacrylique et d'acrylate d'éthyle (type C), notamment celle contenant 30 % de copolymère sec, 0,7 % de lauryl sulfate de sodium et 2,3 % de Polysorbate 80 NF, et commercialisée sous la dénomination Eudragit L30 D55® (Rohm et Haas),
- un copolymère d'acide acrylique et d'ester méthacrylique (type A), notamment celui commercialisé sous la dénomination Eudragit RL 100® (Rohm et Haas).

Parmi les dérivés de la polyvinylpyrrolidone, on pourra citer :
- une povidone, dont le poids moléculaire est de l'ordre de 360 000, commercialisée sous la dénomination Kollidon 90®
- le copolymère polyvinylpyrrolidone / acétate de vinyle 64, de formule (C₆H₉NO)ₙ x (C₄H₆O₂)ₘ dont le poids moléculaire est : (111,1)ₙ x (86,1)ₘ.
- les homopolymères d'alcool polyvinylique.

### Les autre excipients de la composition

Les compositions hormonales topiques à effet systémique selon l'invention peuvent contenir en outre d'autres excipients qui sont des agents complexants, des agents neutralisants tels que l'édétate disodique (EDTA), des agents neutralisants tels que la triéthanolamine (TEA) et/ou des agents plastifiants tels que le phtalate de diéthyle, la triacétine.

Une composition hormonale topique selon l'invention particulièrement appropriée est une composition qui se trouve sous forme de gel et qui contient notamment, dans un mélange hydroalcoolique, 0,4% d'acétate de nomegestrol, 0,15% d'estradiol, 8% de propylèneglycol, 3% d'isopropylidèneglycérol et 2% de silicone DC Blend 20.

De préférence, les compositions conformes à l'invention présentent un pH compris entre 6 et 7 et une viscosité comprise entre 1000 et 2000 mPas.

L'invention concerne également les procédés de préparation des compositions estroprogestatives topiques à effet systémique.
- Un procédé de préparation des compositions sous forme de gel est donné ci-dessous à titre d'exemple.
Les étapes importantes de la préparation d'un gel sont la dispersion de l'agent gélifiant dans l'agent solubilisant (dispersion dont dépendra beaucoup la qualité de la préparation obtenue), l'agitation, l'hydratation, le gonflement et enfin la gélification.

### - Dispersion et agitation : mouillage

L'agent gélifiant (dérivé acrylique) est mis en suspension sous agitation dans le solvant (agent solubilisant). L'agitation doit être modérée car le polymère acrylique perd son pouvoir gélifiant si le cisaillement est trop important.

### - Hydratation et gonflement des polymères

Afin d'éviter la formation d'agglomérats partiellement hydratés, il est recommandé d'incorporer les polymères en les tamisant, pour faciliter la mouillabilité et l'hydratation de la poudre et leur permettre de se déployer en réseau. On favorise cette étape en réalisant un mouillage de la poudre au préalable dans le solvant ou dans le solvant le plus polaire si un système solvant est utilisé.

### - Gélification : neutralisation de la dispersion obtenue

Le pH de la suspension obtenue lors de l'étape précédente est proche de 3 (ce pH est fonction de la concentration en polymère, donc en groupements carboxyliques). On neutralise le milieu en utilisant des bases minérales comme les hydroxydes de sodium, de potassium ou d'ammonium lorsque les solvants de la formulation sont aqueux et des bases organiques comme des amines (triéthanolamine, trométhamine ou TRIS etc.) lorsqu'ils sont peu ou pas polaires. L'ajout de ces agents provoque un épaississement spontané par formation des sels de polymères, solubles dans l'eau.

Un exemple plus particulier de préparation d'un gel conforme à l'invention dont l'agent gélifiant est un dérivé acrylique, se définit en ce que :
- on solubilise les principes actifs et l'EDTA dans le système solvant eau / éthanol à 95° / propylèneglycol en agitant à 300 t/min (30 min environ) ;
- on disperse le polymère acrylique par petites fractions dans la solution de principes actifs en agitant à 100 t/min ;
- on laisse gonfler le polymère acrylique pendant 2 heures sous agitation à 200 t/min;
- on neutralise la dispersion par la triéthanolamine (TEA) dissoute dans une fraction d'eau prélevée sur la quantité à incorporer dans la formulation ; l'agitation est réduite à 100 t/min durant la neutralisation pour éviter l'incorporation de bulles d'air ;
- on agite 30 min à 50 t/min pour homogénéiser le gel obtenu.

• On peut également envisager de préparer des compositions conformes à l'invention sous forme de gels filmogènes (ou gels filmants) et solutions filmogènes (ou solutions filmantes).
De telles formes sont envisagées, car, lors de leur application sur la peau, elles forment, après séchage, un film occlusif suffisant pour augmenter l'hydratation de la peau et améliorer la diffusion des principes actifs qu'elles contiennent. La forme obtenue doit néanmoins pénétrer ou sécher rapidement tout en laissant un toucher agréable et non collant.
Ainsi, un exemple de préparation d'une « solution filmogène » dont l'agent filmogène est solide, se définit en ce que :
- on agite les quantités d'éthanol, d'eau et de propylèneglycol nécessaires à la formulation à 250 t/min pendant 10 min ;
- on solubilise l'EDTA et les principes actifs dans le mélange obtenu ;
- on ajoute l'agent plastifiant et on agite à 250 t/min pendant 30 min ;
- on disperse l'agent filmogène par petites fractions en gardant la même agitation, jusqu'à sa complète solubilisation ; on poursuit l'agitation 1 heure ;
- on ajuste le pH à l'aide d'une solution de triéthanolamine (TEA) dissoute dans une petite quantité d'eau, prélevée sur la quantité d'eau à incorporer à la formulation, en réduisant l'agitation à 100 t/min ; on homogénéise la solution obtenue pendant 30 min.

Un autre exemple de préparation d'une « solution filmogène» dont l'agent filmogène est en dispersion aqueuse, se définit en ce que :
- on mélange à 250 t/min l'eau et un agent plastifiant ; on agite pendant 30 min ;
- on ajoute par petites fractions la dispersion d'agent filmogène en agitant à 250 t/min, jusqu'à obtention d'une solution homogène ; on poursuit l'agitation 1 heure ;
- indépendamment, on solubilise l'EDTA et les principes actifs dans le mélange éthanol et propylèneglycol ; on agite jusqu'à totale dissolution ;
- on ajoute par petites fractions la solution alcoolique de principes actifs dans la solution aqueuse, sous agitation de 250 t/min ; on agite la solution obtenue pendant 1 heure pour l'homogénéiser ;
- on neutralise la solution par la triéthanolamine (TEA) dissoute dans l'eau, en réduisant l'agitation ; on homogénéise la solution obtenue pendant 30 min.

Les gels filmogènes sont obtenus par gélification des solutions filmogènes. On commence ainsi par préparer séparément deux solutions :
- une solution aqueuse renfermant un plastifiant solubilisé, dans laquelle on solubilise totalement l'agent filmogène sous vive agitation;
- une solution alcoolique contenant les autres excipients de la formulation et dans laquelle on solubilise les principe actifs ; on disperse et on laisse gonfler l'agent gélifiant ;
- on mélange la solution alcoolique dans la solution aqueuse et on gélifie la solution par la triéthanolamine.

Les compositions topiques conformes à l'invention sont destinées à être appliquées sur la peau généralement au niveau de l'abdomen, des bras, des cuisses, des fesses....

### PARTIE EXPERIMENTALE

### 1) Evaluation in vitro du passage percutané des principes actifs progestatif/estrogène

Dans cette partie, on étudiera plus particulièrement le passage percutané *in vitro* de l'acétate de nomegestrol et de l'estradiol. Précédemment un gel qui ne contenait que du nomégestrol acétate avait été étudié et avait permis d'obtenir chez la femme des taux circulants suffisants pour assurer un effet endomètrial objectivé par des saignements. Cet essai est décrit dans le document FR 98.03533 au nom du Demandeur. Néanmoins, pour avoir une garantie d'efficacité chez toutes les femmes, il a paru intéressant d'augmenter le flux percutané d'acétate de nomegestrol dans un système capable d'assurer également un flux percutané adéquat d'estradiol.

### a) Principe

L'absorption percutanée est étudiée *in vitro* sur des cellules de diffusion statiques, dites cellules de Franz™. Le passage percutané des principes actifs est évalué par mesure de la radioactivité en utilisant des molécules marquées (carbone 14, tritium). La quantité globale de principe actif absorbé est dosée, à différents temps, dans le compartiment récepteur.

### b) Description de la cellule

Une biopsie cutanée est maintenue horizontalement entre les deux parties de la cellule délimitant ainsi de part et d'autre de l'échantillon, deux compartiments : l'un épidermique, est constitué par un cylindre de verre, de surface définie (1,76 cm²), déposé sur la surface supérieure de la peau ; l'autre dermique (ou récepteur), sur la face inférieure de la biopsie, comprend un réservoir de volume fixe porteur d'un ajutage latéral. Les deux compartiments sont maintenus en place par l'intermédiaire d'un clamp.
Le compartiment récepteur est rempli d'un liquide de survie constitué d'une solution de tampon phosphate isotonique à pH 7,4 (Phosphate monosodique : 0,04 M ; Phosphate disodique : 0,02 M; NaCl : 0,08 M, Albumine : 15g/l). Des prélèvements du liquide contenu dans le compartiment inférieur sont effectués par l'ajutage latéral au cours de l'expérience et au terme de l'essai. Pour chaque temps, le liquide de survie est prélevé en totalité et remplacé par du liquide neuf. Une jaquette à double circulation d'eau entoure la partie inférieure de la cellule et permet de thermostater l'ensemble à une température de 37°C. L'homogénéité du contenu du compartiment récepteur est assuré par les mouvements d'un barreau magnétique. La partie supérieure de la cellule est ouverte vers l'extérieur exposant ainsi la surface de l'épiderme à l'air ambiant du laboratoire comme dans le cas d'une application *in vivo.*

### c) Préparation des biopsies cutanées

La peau, d'origine humaine, a été prélevée chez le sujet sain, au niveau abdominal, au cours d'une intervention de chirurgie plastique. Les prélèvements cutanés sont débarrassés des graisses sous cutanées adhérentes à l'aide d'un scalpel.

### d) Mise en oeuvre de la méthode

Les préparations à étudier sont appliquées en quantités pesées, à l'aide d'une spatule sur toute la surface épidermique circonscrite par le cône de verre. A intervalles de temps précis (2h, 4h, 6h, 8h, 10h, 24h), la totalité du liquide contenu dans le compartiment récepteur est prélevée par l'ajutage latéral et remplacé par du liquide neuf. Les échantillons prélevés sont traités avec du liquide de scintillation Pico-fluor 40™ et l'activité mesurée à l'aide du compteur à scintillation liquide.

### e) Détermination de la radioactivité

La scintillation en milieu liquide a été utilisée pour détecter les rayonnements β émis par les radio-éléments. Les mesures sont effectuées au moyen d'un compteur à scintillation liquide Packard Tricarb 4435™.

### f) Expression des résultats

Le passage percutané des principes actifs est évalué à chaque temps de prélèvement par la mesure de la quantité cumulée (µg) de principe actif en fonction du temps.

Les résultats moyens correspondent à quatre déterminations expérimentales par formule et sont associés à l'écart-type (E.T.).

### 2) Exemple 1 : 1^{ère} série de gels

### Les formulations testées

Le passage percutané des 2 principes actifs a été étudié *in vitro* pour les gels dont les formules figurent au tableau 1 (Tableau 1). Ces gels diffèrent par leur teneur en estradiol, leur teneur en agent solubilisant, la nature et la teneur en agents promoteurs de passage et le rapport entre agents solubilisants et agents promoteurs de passage. Pour cette 1^{ère} série de gels, la concentration en acétate de nomegestrol dans la formulation est fixée à 0,4%.

**Tableau 1 : Composition en pourcentage des gels de la 1^{ère} série**

| **REFERENCE** | **G29-287** | **G29-299** | **Tx11323 lot-12** | **G42-110** | **G42-111** | **G47-1** | **G47-5** |
|---|---|---|---|---|---|---|---|
| AcN (acétate de nomegestrol) | 0,4 | 0,4 | - | 0,4 | 0,4 | 0,4 | 0,4 |
| Estradiol | - | 0,1 | 0,1 | 0,15 | 0,1 | 0,2 | 0,15 |
| Carbopol 1342 ou 1382 | 0,5 | 0,5 | 0,5 | 0,5 | 0,5 | 0,5 | 0,5 |
| Propylèneglycol | 6 | 6 | 6 | 8 | 8 | 8 | 6 |
| Transcutol | 5 | 5 | 5 | - | - | - | - |
| Solkétal | | | | 3 | 3 | 3 | 5 |
| EDTA | 0,05 | 0,05 | 0,05 | 0,05 | 0,05 | 0,05 | 0,05 |
| Triéthanolamine | 0,3 | 0,3 | 0,3 | 0,3 | 0,3 | 0,3 | 0,3 |
| Eau déminéralisée | 42,75 | 42,65 | 43,05 | 42,6 | 42,65 | 42,55 | 42,6 |
| Ethanol à 95° | 45 | 45 | 45 | 45 | 45 | 45 | 45 |

Les principales différences dans la composition de ces gels résident dans :
- les proportions Acétate de Nomegestrol (AcN)/ Estradiol (E2) utilisées,
- le choix de l'agent promoteur d'absorption (« enhancer »), à savoir Solketal ou Transcutol,
- les proportions respectives PropylèneGlycol (PG)/Solkétal,

Toutes ces formulations de gels répondent aux spécifications de pH, viscosité, titre et aspect. Elles sont testées en passage percutané.

### Résultats de passage percutané in vitro

Le tableau 2 ci-dessous résume les quantités cumulées (µg) d'acétate de nomegestrol et d'estradiol retrouvées dans le compartiment récepteur en 24 heures.

**Tableau 2 : Résultats des études de passage cutané in vitro sur les gels de la 1^{ère} série**

| | | **G29-287** | **G29-299** | **Tx11323 lot-12** | **G42-110** | **G42-111** | **G42-112** | **G47-1** | **G47-5** |
|---|---|---|---|---|---|---|---|---|---|
| AcN (µg) | Moy. | 1,253 | 1,741 | - | 2,299 | 2,148 | 1,407 | 1,816 | 1,785 |
| | E.T | 0,791 | 1,216 | - | 0,524 | 1,535 | 1,021 | 1,151 | 0,523 |
| E2 (µg) | Moy. | - | 0,680 | 1,396 | 1,121 | 0,658 | 0,381 | 0,909 | 0,913 |
| | E.T. | - | 0,149 | 0,653 | 0,257 | 0,469 | 0,276 | 0,614 | 0,258 |

### 3) Influence de la présence de l'estradiol sur la diffusion de l'acétate de nomegestrol

**Tableau 3 : Comparaison des résultats de passage cutané des gels G29-287, G29-299 et TX11323**

| | | **G29-287** | **G29-299** | **Tx11323 lot-12** |
|---|---|---|---|---|
| AcN (µg) | Moy. | 1,253 | 1,741 | - |
| | E.T. | 0,791 | 1,216 | - |
| E2 (µg) | Moy. | - | 0,680 | 1,396 |
| | E.T.. | - | 0,149 | 0,653 |

Les gels G29-287, G29-299 et TX11323 différent uniquement par la nature du ou des principe(s) actif(s) qu'ils renferment, le reste de leur composition étant strictement identique.
Le gel G29-287 contient uniquement de l'acétate de nomegestrol à 0,40%, le gel TX11323 contient uniquement de l'estradiol à 0,10 % et le gel G29-299 contient l'association des deux principes actifs, acétate de nomegestrol et estradiol, respectivement à 0,40 % et 0,10 %.
La meilleure diffusion de l'acétate de nomegestrol vers le compartiment récepteur est obtenue pour le gel G29-299, c'est à dire pour le gel contenant de l'acétate de nomegestrol associé à de l'estradiol : 1,741 µg d'acétate de nomegestrol cumulé pour le gel G29-299 contre 1,253 µg d'acétate de nomegestrol cumulé pour le gel G29-287, c'est-à-dire pour le gel contenant uniquement de l'acétate de nomegestrol.
La meilleure diffusion de l'estradiol est obtenue pour le gel TX11323 (1,396 µg en 24 heures).
Ce gel ne contient pas d'acétate de nomegestrol, mais uniquement de l'estradiol.
On peut en conclure que l'estradiol favorise le passage de l'acétate de nomegestrol (+ 39 %) tandis que l'acétate de nomegestrol s'oppose partiellement à la pénétration percutanée de l'estradiol (- 51 %).

### 4) Influence de la concentration de l'estradiol sur la diffusion de l'acétate de nomegestrol

Afin de vérifier si l'effet promoteur de l'estradiol sur la diffusion de l'acétate de nomegestrol était dépendant de la concentration de l'estrogène, on a comparé, en termes de passage percutané, les formulations G42-111 contenant 0,10 % d'estradiol, G42-110 contenant 0,15 % d'estradiol et G47-1 contenant 0,20 % d'estradiol. Ces formulations ne diffèrent que par leur teneur en estradiol.
Les résultats de passage percutané *in vitro* sont résumés dans le tableau 4 :

**Tableau 4 : Comparaison des résultats de passage cutané des gels G42-111, G42-110 et G47-1**

| | | **G42-111** | **G42-110** | **G47-1** |
|---|---|---|---|---|
| AcN (µg) | Moy. | 2,148 | 2,299 | 1,816 |
| | E.T. | 1,535 | 0,524 | 1,151 |
| E2 (µg) | Moy. | 0,586 | 1,121 | 0,909 |
| | E.T. | 0,469 | 0,257 | 0,614 |

La meilleure diffusion de l'acétate de nomegestrol dans le compartiment récepteur est obtenue pour le gel G42-110, c'est-à-dire pour le gel contenant 0,15 % d'estradiol : la quantité cumulée d'acétate de nomegestrol est de 2,299 µg pour le gel G42-110 (0,15% E2) contre 2,148 µg pour le gel G42-111 (0,10% E2) et 1,816 µg pour le gel G47-1 (0,20% E2).

La meilleure diffusion de l'estradiol est obtenue avec le gel G42-110 (1,121 µg) qui donne de meilleurs résultats que le gel G47-1 (0,909 µg) qui lui même donne de meilleurs résultats que le gel G42-111 (0,586 µg). La diffusion percutanée de l'estradiol à partir de gels contenant également de l'acétate de nomegestrol n'est donc pas proportionnelle à la concentration d'estradiol. Ainsi, les quantités d'estradiol pouvant diffuser vers le compartiment récepteur à partir du gel à 0,15% sont supérieures à celles qui diffusent à partir des gels à 0,10 % et à 0,20 % d'estradiol.

### Conclusion :

D'une manière surprenante, on observe avec les formulations dans lesquelles l'acétate de nomégestrol est associé à l'estradiol, un effet promoteur de l'estradiol sur la diffusion de l'acétate de nomegestrol. Cet effet promoteur est mis en évidence avec deux couples de solvants différents : le couple propylène-glycol / Transcutol (tableau 3) et le couple propylène glycol / Solkétal (tableau 4). De façon surprenante également, il est démontré que l'effet promoteur exercé par l'estradiol sur la diffusion de l'acétate de nomegestrol n'est pas proportionnel à la concentration en estradiol et qu'il est maximal pour une concentration en estradiol de l'ordre de 0,15 %. A l'inverse, la présence d'acétate de nomegestrol dans la formulation freine la diffusion de l'estradiol dans le compartiment récepteur par rapport à une formulation de gel contenant uniquement de l'estradiol.

### 5) Résultats observés in vivo

Le facteur limitant, en terme de passage étant l'acétate de nomegestrol, un essai de pharmacocinétique a été réalisée chez la femme avec 2 gels contenant 0,4% d'acétate de nomegestrol et prenant en compte les résultats précédemment obtenus *in vitro.* Ces 2 gels, qui ne différaient que par le type de Carbopol utilisé, avaient les compositions en pourcentage suivantes :

**Tableau 5 : formule des 2 gels utilisés pour des études de pharmacocinétique chez la femme**

| | Gel A | Gel B |
|---|---|---|
| Acétate de nomegestrol | 0,40 | 0,40 |
| Propylène glycol | 8,00 | 8,00 |
| Solkétal | 3,00 | 3,00 |
| Carbopol 980 | 0,60 | |
| Carbopol 1382 | | 0,50 |
| EDTA | 0,05 | 0,05 |
| TEA | 0,24 | 0,30 |
| Ethanol à 95° | 45,00 | 45,00 |
| Eau déminéralisée | 42,69 | 42,73 |

Après 3 jours d'administration chez 6 femmes ménopausées volontaires (3 g de gel chaque jour, étendus sur 400 cm²), les taux plasmatiques moyens étaient égaux à :

| | |
|---|---|
| Gel A | 0,345 ng/ml |
| Gel B | 0,456 ng/ml |

Les données cinétiques montrent que l'état d'équilibre se situe aux alentours de 0,7 ng/ml. La connaissance de la pharmacocinétique de l'acétate de nomegestrol permet d'affirmer que l'efficacité clinique est obtenue pour des concentrations plasmatiques égales ou supérieures à 0,7 ng/ml. Des taux plasmatiques de l'ordre de 0,7 ng sont suffisants pour observer un effet endométrial objectivé par des saignements (ces résultats sont décrits dans le brevet FR 2.776.191 appartenant au Demandeur).Les formulations A et B répondent donc aux objectifs. Cependant, pour assurer un effet thérapeutique complet chez toutes les femmes il pouvait être intéressant d'obtenir des taux circulants plus élevés d'acétate de nomegestrol. De façon inattendue, les résultats de diffusion *in vitro* ont encore pu être améliorés par la mise au point d'autres formulations.

Le gel B avait précédemment été testé *in vitro* selon la même méthode que celle utilisée dans l'étude des gels en association. Les résultats de passage percutané à 24 heures sont présentés dans le tableau 6 :

**Tableau 6 : Résultats de passage cutané sur la formulation type Gel B**

| | | **Gel B** |
|---|---|---|
| AcN (µg) | Moy. | 1,662 |
| | E.T. | 0,649 |

Ainsi, *in vitro* et dans les conditions opératoires utilisées pour ces essais, une diffusion dans le compartiment récepteur de 1,66 µg d'AcN permet d'obtenir un taux plasmatique à l'équilibre de l'ordre de 0,7 ng/ml.
Les gels d'acétate de nomegestrol susceptibles de donner des résultats cliniques satisfaisants doivent donc présenter, lors des essais de passage percutané *in vitro,* des quantités cumulées d'acétate de nomégestrol à 24 heures supérieures à 1,66 µg.

De la même façon, le gel TX11323, appliqué à raison de 3 g de gel sur une surface corporelle de 400 cm² conduit à des taux plasmatiques d'estradiol à l'équilibre d'environ 40 pg/ml, ce qui se situe dans la zone des concentrations plasmatiques d'estradiol efficaces puisque celles-ci sont comprise entre 30 et 60 ng/ml.
Ainsi, *in vitro,* une diffusion dans le compartiment récepteur de 1,40 µg en 24 heures (tab 2), conduit à des taux plasmatiques d'estradiol à l'équilibre, de l'ordre de 40 pg/ml. Les gels d'estradiol aptes à donner des résultats cliniques satisfaisants doivent donc présenter lors des essais de passage percutané, des quantités cumulées d'estradiol à 24 heures supérieures à 1,05 µg sans cependant dépasser 2,1 µg afin de ne pas induire d'hyperestrogénie.

Une nouvelle série de gels a donc été fabriquée afin de confirmer les premiers résultats obtenus, et notamment l'effet promoteur exercé par l'estradiol sur l'absorption cutanée de l'acétate de nomegestrol et d'étudier l'action combinée d'autres promoteurs d'absorption.

### 6) Etude d'autres familles de promoteurs d'absorption (2^{ème} série de gels)

Le passage percutané des deux principes actifs a été étudié *in vitro* dans des formulations de gels ne différant que par la nature du promoteur d'absorption utilisé, les autres éléments de la formulation restant identiques :

**Tableau 7 : Formulation type des gels contenant d'autres familles de promoteurs d'absorption (2^{ème} série de gels).**

| Constituants | Pourcentage dans la formule |
|---|---|
| Acétate de nomegestrol (AcN) | 0,42 |
| Estradiol | 0,15 |
| Carbopol 1382 | 0,50 |
| Propylène Glycol | 8,0 |
| Promoteur d'absorption | **3,00** |
| EDTA | 0,05 |
| TEA | 0,30 |
| Ethanol à 95° | 45,00 |
| Eau déminéralisée | 42,58 |

### Les formulations correspondant à cette formulation de base sont résumées ci-dessous :

**Tableau 8 : Promoteurs d'absorption testés.**

| Famille chimique | Dénomination chimique | Dénomination commerciale | Réf. Formulation |
|---|---|---|---|
| | Monocaprylate de propylène glycol | Capryol 90 | G49-80 |
| Polyols | PEG-6 caprylic/capric glycérides | Softigen 767 | G49-88 |
| | PEG-7 glycéryl cocoate | Cetiol HE | G49-102 |
| | PPG-5 ceteth-20 | Procetyl AWS | G49-110 |
| Acide gras | Acide octanoïque | Acide Caprylique | G49-100 |
| | Acide octadécènoïque-9 | Acide Oleique | G49-106 |
| | Acide dodécanoïque | Acide Laurique | G49-108 |
| Esters d'acide gras | Adipate de diisopropyle | Crodamol DA | G49-90 |
| Surfactifs | Polysorbate 80 | Montanox 80 | G49-82 |
| | Dicaprylate/caprate de propylène glycol | Mygliol 840 | G49-111 |
| Alcools | Octyl-2 dodécanol | Eutanol G | G49-104 |
| Amides | 1-dodécylazacyclohepta-2-one | Azone | G49-139 |

Dans une autre formulation, référencée G42-120, on a ajouté au gel référencé G42-110 (Solketal 3%) une silicone, le DC Blend 20, à raison de 2 % en masse de la formulation. Ces formulations ont également été comparées à une formulation témoin (formule identique mais ne contenant pas d'agent promoteur d'absorption : Réf. G49-114).
La formule correspondant à ce gel est présentée dans le tableau 9.

**Tableau 9 : Composition du gel G49-114**

| Constituants | Référence G49-114 (témoin) |
|---|---|
| Acétate de nomegestrol | 0,42 |
| Estradiol | 0,15 |
| Carbopol 1382 | 0,50 |
| Propylène Glycol | 8,00 |
| Promoteur d'absorption | - |
| EDTA | 0,05 |
| TEA | 0,30 |
| Ethanol à 95° | 45,00 |
| Eau déminéralisée | 45,58 |

### Résultats in vitro :

Les résultats *in vitro* obtenus avec ces formulations et exprimés en quantités cumulées de principes actifs ayant diffusé dans le compartiment récepteur en 24 heures, sont résumés dans les tableaux 10a et 10b:

### Tableaux 10a et 10b : Résultats des études de passage cutané réalisées sur les gels de la 2^{ème} série

**Tableau 10a**

| | | G49-114 | G49-80 | G49-88 | G49-102 | G49-110 | G49-100 | G49-106 | G49-108 |
|---|---|---|---|---|---|---|---|---|---|
| AcN (µg) | Moy. | 1,368 | 1,468 | 1,400 | 1,053 | 0,576 | 3,820 | 1,988 | 3,761 |
| | E.T. | 0,508 | 0,646 | 0,307 | 0,527 | 0,102 | 1,179 | 0,814 | 0,437 |
| E2 (µg) | Moy. | 0,698 | 0,732 | 0,984 | 0,307 | 0,13 | 1,91 | 1,527 | 2,308 |
| | E.T. | 0,247 | 0,320 | 0,220 | 0,181 | 0,029 | 0,503 | 0,554 | 0,304 |

**Tableau 10b**

| | | G49-82 | G49-111 | G49-104 | G49-139 | G49-90 | G42-120 |
|---|---|---|---|---|---|---|---|
| AcN (µg) | Moy. | 1,099 | 1,572 | 1,514 | 1,352 | 0,788 | 2,782 |
| | E.T. | 0,310 | 0,358 | 0,548 | 0,465 | 0,178 | 1,068 |
| E2 (µg) | Moy. | 0,303 | 1,217 | 0,700 | 0,173 | 0,235 | 1,368 |
| | E.T. | 0,104 | 0,313 | 0,318 | 0,044 | 0,124 | 0,562 |

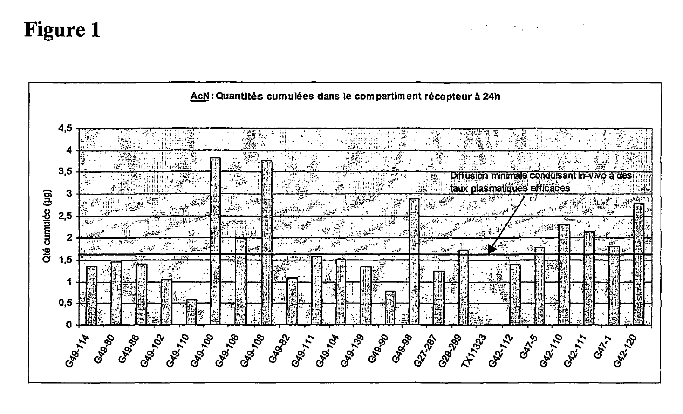

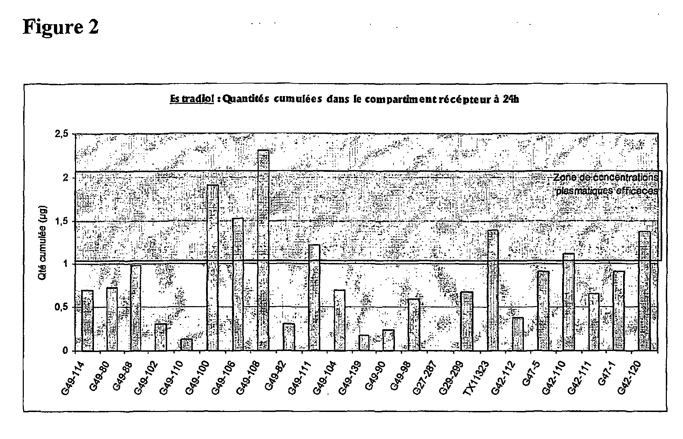

L'analyse des résultats représentés dans les figures 1 et 2 montre que l'on peut ajuster les quantités d'acétate de nomegestrol et d'estradiol diffusibles, par le choix de la nature du promoteur d'absorption utilisé. Lorsque l'on compare les quantités de principes actifs ayant diffusé dans le compartiment récepteur à partir des formulations contenant un promoteur d'absorption, à celles observées avec la formulation témoin, on constate que certains promoteurs favorisent de manière exclusive la diffusion de l'estradiol sans affecter la diffusion de l'acétate de nomegestrol. Ce phénomène est observé avec le Softigen 767 (G49-88) et le Miglyol 840 (G49-111). D'autres promoteurs favorisent de façon simultanée le passage des deux actifs. C'est le cas des acides gras à chaîne longue (G49-100, G49-106 et G49-108), et des formulations G42-110 et G42-120 à base de Solkétal. Aucun des promoteurs actuellement étudiés n'est capable de promouvoir exclusivement le passage de l'acétate de nomegestrol.
De façon inattendue, on a observé que certains excipients, classiquement décrits comme promoteurs d'absorption, au contraire freinent la diffusion du (des) principe(s) actif(s). Ainsi, l'Azone, (gel référencé G49-139), réduit significativement la diffusion de l'estradiol vers le compartiment récepteur et reste sans effet sur la diffusion de l'acétate de nomegestrol. Le Cetiol HE (G49-102), le Procétyl AWS (G49-110), le Montanox 80 (G49-82) et le Crodamol DA (G49-90) provoquent quant à eux une diminution importante de la diffusion des deux principes actifs par rapport à la formulation ne contenant pas de promoteur d'absorption. Ainsi, la nature de l'agent promoteur d'absorption susceptible d'améliorer le passage simultané de l'acétate de nomegestrol et de l'estradiol ne paraît pas pouvoir être prévue. Néanmoins, en fonction des corrélations *in vitro* / *in-vivo* préalablement établies, on constate que certaines formulations permettent d'assurer à la fois un taux plasmatique efficace d'acétate de nomegestrol et un taux plasmatique efficace d'estradiol et ce, malgré les compétitions précédemment décrites en terme de passage percutané entre les deux molécules. Il s'agit notamment des formulations référencées G42-120, G49-106, G49-108 et G49-100. Certaines de ces formulations contiennent des promoteurs connus pour être assez mal tolérés par la peau et devront être utilisées avec précaution. C'est pourquoi la formulation G42-120, qui contient un agent filmogène associé à d'autres excipients dont l'innocuité est bien démontrée, sera celle qui sera privilégiée.

### 7) Rôle de l'agent filmogène

**Tableau 11 : Comparaison des résultats de passage cutané des gels G42-110 et G42-120**

| | | **G42-110** | **G42-120** |
|---|---|---|---|
| AcN (µg) | Moy. | 2,299 | 2,782 |
| | E.T. | 0,524 | 1,068 |
| E2 (µg) | Moy. | 1,121 | 1,368 |
| | E.T. | 0,257 | 0,562 |

La seule différence entre les gels G42-110 et G42-120 réside dans l'ajout d'un agent filmogène (silicone DC Blend 20) dans le second. Ces 2 gels contiennent 8 % de Propylène glycol (PG) et 3 % de Solkétal (S).

La diffusion de l'acétate de nomegestrol la plus forte est obtenue pour le gel G42-120 : la quantité cumulée d'acétate de nomegestrol ayant diffusé sur une période de 24 h, est de 2,782 µg pour le gel G42-120 contre 2,299 µg pour le gel G42-110, ce qui représente une augmentation de plus de 20 % de la diffusion d'acétate de nomegestrol.
De même, l'estradiol diffuse mieux au travers de la peau avec le gel G42-120 : la quantité cumulée d'estradiol, sur une période de 24 h, est de 1,368 µg pour le gel G42-120 contre 1,121 µg pour le gel G42-110, ce qui représente également une augmentation de plus de 20 % de la diffusion d'estradiol.

### 8) Influence de la concentration de l'acétate de nomégestrol sur la diffusion in vitro

Tous les essais précédents ayant été faits sur des formulations à 0,4% d'acétate de nomégestrol, on a voulu étudier l'influence sur la diffusion percutanée de la concentration de l'acétate de nomégestrol dans le gel. Etant donné les bons résultats obtenus avec le gel G42-110 dans les études de passage précédentes, on a utilisé cette formulation comme point de départ. Afin de permettre la dissolution de quantités plus importantes d'acétate de nomégestrol, on a augmenté les concentrations de Solkétal et d'alcool qui sont d'excellents solvants pour ce principe actif. Enfin, pour maintenir la même viscosité avec ces gels par rapport au gel G42-110, la concentration du Carbopol a également été augmentée. Les formulations sont présentées dans le tableau 12.

**Tableau 12 : Composition en pourcentage des gels**

| | G42-110 | G52-289 | G52-290 |
|---|---|---|---|
| AcN | 0,4 | 1,00 | 1,7 |
| Estradiol | 0,15 | 0,15 | 0,15 |
| Carbopol 1382 | 0,5 | 0,7 | 0,7 |
| Propylène glycol | 8 . | 8 | 8 |
| Solkétal | 3 | 6 | 6 |
| EDTA | 0,05 | 0,05 | 0,05 |
| Triéthanolamine | 0,3 . | 0,3 | 0,3 |
| Eau déminéralisée | 42,6 | 28,8 | 28,1 |
| Ethanol à 95° | 45 | 55 | 55 |

### Résultats de passage percutané in vitro

Le tableau 13 ci-dessous résume les quantités cumulées (µg) d'acétate de nomégestrol retrouvées dans le compartiment récepteur en 24 heures.

**Tableau 13 : Résultats des études de passage cutané in vitro sur les gels à concentrations différentes d'acétate de nomégestrol**

| | G42-110* | G52-289 | G52-290 |
|---|---|---|---|
| Conc AcN | 0,4 | 1,0 | 1,7 |
| AcN(µg) | 2,30* | 2,93 | 4,68 |

| | | | |
|---|---|---|---|
| quantités cumulées (µg) d'acétate de nomégestrol retrouvées dans le compartiment récepteur en 24 heures. | | | |
| * résultat obtenu dans les essais de la 1ère série | | | |

On a pu démontrer précédemment que les gels d'acétate de nomégestrol, susceptibles de donner des résultats cliniques satisfaisants, doivent présenter, lors des essais de passage percutané *in vitro,* des quantités cumulées d'acétate de nomégestrol supérieures à 1,66 µg à 24 heures.

Les résultats du tableau 13 démontrent que, dans une formulation appropriée, des gels possédant des concentrations d'acétate de nomégestrol allant de 0,4 à 1,7 % délivrent des quantités cumulées d'acétate de nomégestrol très supérieures à 1,66 µg à 24 heures et donc sont susceptibles de donner des résultats cliniques satisfaisants. On a constaté également que la quantité de principe actif qui passe à travers la peau augmente en fonction de sa concentration dans le gel.

### 9) Rôle de la proportion « agent solubilisant/agent promoteur »

Le tableau 14 ci-dessous présente les résultats de passage *in vitro* exprimés en quantités cumulées à 24 heures observés sur les formulations G42-110 et G47-5 de la première série de gels à 0,4 % d'acétate de nomégestrol qui ne diffèrent que dans le choix des proportions Propylène glycol (PG)/Solkétal (S), qui sont respectivement de 8/3 pour le gel G42-110 et 6/5 pour le gel G47-5.

**Tableau 14 : Comparaison des résultats de passage cutané des gels G42-110 et G47-5**

| | | **G42-110** | **G47-5** |
|---|---|---|---|
| AcN (µg) | Moy. | 2,299 | 1,785 |
| | E.T. | 0,524 | 0,523 |
| E2 (µg) | Moy. | 1,121 | 0,913 |
| | E.T. | 0,257 | 0,258 |

Le gel G42-110 permet une meilleure diffusion de l'acétate de nomegestrol que le gel G47-5 : on obtient 2,299 µg d'AcN cumulé pour le gel G42-110 contre 1,785 µg pour le gel G47-5. De même, le gel G42-110 permet également une meilleure diffusion de l'estradiol (E2) que le gel G47-5 : on obtient 1,121 µg d'E2 cumulé pour le gel G42-110 contre 0,913 µg pour le gel G47-5. Ainsi, pour ce type de formulation, il est plus avantageux d'utiliser le système PG/S dans les proportions 8/3 que 6/5.

Dans une autre série d'essais, on a étudié l'influence du couple propylène glycol/solkétal sur le passage percutané de gels à plus forte concentration d'acétate de nomégestrol, c'est-à-dire à 1,0 %.

**Tableau 15 : Composition en pourcentage des gels**

| | **G235-001** | **G52-289** | **G52-292** |
|---|---|---|---|
| AcN | 1,00 | 1,00 | 1,00 |
| Estradiol | 0,15 | 0,15 | 0,15 |
| Carbopol 1382 | 0,7 | 0,7 | 0,7 |
| Propylène glycol | 8 | 8 | 3 |
| Solkétal | 3 | 6 | 11 |
| EDTA | 0,05 | 0,05 | 0,05 |
| Triéthanolamine | 0,3 | 0,3 | 0,3 |
| Eau déminéralisée | 31,8 | 28,8 | 28,8 |
| Ethanol à 95° | 55 | 55 | 55 |

Ces formulations ne diffèrent que dans les proportions du mélange propylène glycol/solkétal.

**Tableau 16 : Résultats des études de passage cutané in vitro sur les gels à proportions différentes de propylène glycol/solkétal**

| | **G235-001** | **G52-289** | **G52-292** |
|---|---|---|---|
| Propylène glycol | 8 | 8 | 3 |
| Solkétal | 3 | 6 | 11 |
| AcN (µg) | 3,17 | 2,93 | 1,61 |
| E2 (µg) | 0,71 | 0,64 | 0,32 |

De manière surprenante, on a constaté que, pour ces formulations, l'augmentation de la concentration de l'agent promoteur, le Solkétal, induit une diminution du taux de passage des deux hormones.
Les résultats de ces deux séries d'essais démontrent que pour chaque formulation, la proportion optimale entre l'agent solubilisant et le promoteur d'absorption ne peut être connue d'avance et doit être étudiée cas par cas.

### 10) CONCLUSION

Les essais de cette invention attestent que la concentration de chaque composant et son interaction avec les autres composants d'une formulation de gel cutané peuvent, de manière surprenante, mener à une synergie ou, au contraire, à une inhibition de la quantité des principes actifs qui passent par la voie percutanée. De ce fait, les préparations précédemment décrites pour le passage percutané d'un progestatif de synthèse seul, comme décrit, ne sont pas directement applicables à une association estro-progestative car chaque association de ce type demande une solution galénique particulière.

En particulier :
- l'estradiol, utilisé à certaines concentrations, peut favoriser le passage percutané de l'acétate de nomegestrol ;
- l'acétate de nomegestrol, en général, diminue le passage percutané de l'estradiol ;
- la nature du promoteur d'absorption assurant un passage percutané adéquat pour chaque principe actif n'est pas prévisible ;
- parmi les promoteurs d'absorption, les dioxolanes (tels que le Solkétal et le SEPA) sont ceux qui permettent le mieux d'atteindre les objectifs ;
- le rapport optimal entre le solvant et le promoteur de passage doit être déterminé pour chaque formulation ;
- l'addition d'un agent filmogène comme la silicone DC Blend 20, permet d'augmenter le passage percutané des principes actifs, acétate de nomegestrol et estradiol, d'environ 20% tout en améliorant les caractéristiques cosmétiques de la composition.

## Revendications

1. Composition hormonale topique à effet systémique, pour le traitement hormonal de la périménopause et de la ménopause ainsi que pour le traitement du déficit hormonal ovarien chez la femme en aménorrhée, à base d'un progestatif et d'un agent estrogène, **caractérisée en ce qu'**elle renferme :
- à titre de principes actifs, le nomégestrol et/ou un de ses esters ou un de ses éthers comme progestatif, et un estrogène,
- ainsi qu'un véhicule permettant le passage systémique desdits principes actifs choisi dans le groupe constitué par un agent solubilisant, un agent promoteur d'absorption choisi dans le groupe des dioxolanes, un agent filmogène, un agent gélifiant et leurs mélanges,
en association ou en mélange avec des excipients appropriés pour la réalisation d'une forme pharmaceutique, gélifiée et/ou filmogène.

2. Composition hormonale topique à effet systémique selon la revendication 1, **caractérisée en ce que** le progestatif est l'acétate de nomégestrol.

3. Composition hormonale topique à effet systémique selon la revendication 1 ou la revendication 2, **caractérisée en ce que** l'estrogène est l'estradiol et/ou un de ses esters ou un de ses éthers.

4. Composition hormonale topique à effet systémique selon la revendication 3, **caractérisée en ce que** l'estrogène est un ester d'acide gras d'estradiol, et plus précisément le valérate d'estradiol.

5. Composition hormonale topique à effet systémique selon la revendication 3, **caractérisée en ce que** l'estrogène est un éther d'estradiol, et en particulier le promestriène.

6. Composition hormonale topique à effet systémique selon l'une quelconque des revendications 1 à 5, **caractérisée en ce que** la quantité de nomégestrol ou d'un de ses esters ou éthers varie de 0,05 à 5,0 % en poids de la composition totale.

7. Composition hormonale topique à effet systémique selon la revendication 6, **caractérisée en ce que** la quantité de nomegestrol ou d'un de ses esters ou éthers est de l'ordre de 1,0 % en poids de la composition totale.

8. Composition hormonale topique à effet systémique selon l'une quelconque des revendications 1 à 7, **caractérisée en ce que** la quantité d'estradiol ou d'un de ses esters ou éthers varie de 0,05 à 1,0 % en poids de la composition totale.

9. Composition hormonale topique à effet systémique selon la revendication 8, **caractérisée en ce que** la quantité d'estradiol ou d'un de ses esters ou éthers est de l'ordre de 0,15 % en poids de la composition totale.

10. Composition hormonale topique à effet systémique selon l'une quelconque des revendications 1 à 9, **caractérisée en ce que** l'agent solubilisant est choisi dans le groupe constitué par les alcools, les mélanges hydroalcooliques, le propylèneglycol et leurs mélanges.

11. Composition hormonale topique à effet systémique selon l'une quelconque des revendications 1 à 10, **caractérisée en ce que** l'agent solubilisant est un mélange ternaire éthanol / eau / propylèneglycol, dans lequel la quantité d'éthanol varie de 30 à 60 % en poids de la composition totale, la quantité d'eau varie de 20 à 60 % en poids de la composition totale et celle de propylèneglycol de 2 à 20 % en poids de la composition totale.

12. Composition hormonale topique à effet systémique selon la revendication 1 à 11, **caractérisée en ce que** la quantité d'agent promoteur d'absorption varie de 2 à 12 % en poids de la composition totale.

13. Composition hormonale topique à effet systémique selon la revendication 1 à 12, **caractérisée en ce que** l'agent promoteur d'absorption est l'isopropylidèneglycérol ou le 2n-nonyl 1,3-dioxolane.

14. Composition hormonale topique à effet systémique selon l'une quelconque des revendications 1 à 13, **caractérisée en ce que** l'agent gélifiant est choisi dans le groupe constitué par les dérivés cellulosiques et les dérivés acryliques.

15. Composition hormonale topique à effet systémique selon la revendication 14, **caractérisée en ce que** le dérivé acrylique est un carbomère utilisé à une concentration allant de 0,3 à 1% en poids de la composition totale.

16. Composition hormonale topique à effet systémique selon l'une quelconque des revendications 1 à 15, **caractérisée en ce que** l'agent filmogène est choisi dans le groupe constitué par les silicones, les dérivés cellulosiques, les dérivés méthacryliques et les dérivés de la polyvinylpyrrolidone.

17. Composition hormonale topique à effet systémique selon la revendication 16, **caractérisée en ce que** la silicone est choisie dans le groupe constitué par la diméthicone, le diméthiconol, la simethicone et leurs mélanges.

18. Composition hormonale topique à effet systémique selon la revendication 17, **caractérisée en ce que** la silicone est le mélange formé de diméthiconol dans de la diméthicone.

19. Composition hormonale topique à effet systémique selon la revendication 17 ou 18, **caractérisée** la quantité de silicone utilisée varie de 1 à 3 % en poids de la composition totale.

20. Composition hormonale topique selon l'une quelconque des revendications 1 à 19, sous forme de gel pénétrant, **caractérisée en ce qu'**elle contient, dans un mélange hydroalcoolique, 0,4 % d'acétate de nomegestrol, 0,15 % d'estradiol, 8 % de propylèneglycol, 3 % d'isopropylidèneglycérol et 2 % de mélange formé de diméthiconol dans de la diméthicone.

21. Composition hormonale topique selon l'une quelconque des revendications 1 à 19, **caractérisée en ce qu'**elle contient, sous forme de gel pénétrant, un mélange hydroalcoolique, 0,4 % d'acétate de nomegestrol, 0,15 % d'estradiol, 8 % environ de propylèneglycol et 3 % environ d'isopropylidèneglycérol.

## Claims

1. Topical hormonal compositions with a systemic effect, for the hormonal treatment of the perimenopause and of the menopause as well as for the treatment of ovarian hormonal deficiencies in women with amenorrhea, based on a progestogen and an oestrogenic agent, **characterized in that** they contain:
- as active ingredients, nomegestrol and/or one of its esters or one of its ethers as a progestogen, and an oestrogen,
- as well as a vehicle allowing the systemic passage of said active ingredients selected from the group consisting of a solubilizing agent, an absorption promoting agentselected among the dioxolanes, a film-forming agent, a gelling agent and their mixtures,
in combination or in admixture with suitable carriers for the achievement of a pharmaceutical, gelled and/or film-forming form.

2. Topical hormonal compositions with a systemic effect according to claim 1 or claim 2, **characterized in that** the progestogen is nomegestrol acetate.

3. Topical hormonal compositions with a systemic effect according to claim 1 or claim 2, **characterized in that** the oestrogen is estradiol and/or one of its esters or its ethers.

4. Topical hormonal compositions with a systemic effect according to claim 3, **characterized in that** the oestrogen is a fatty acid ester of estradiol, more specifically estradiol valerate.

5. Topical hormonal compositions with a systemic effect according to claim 3, **characterized in that** the oestrogen is an estradiol ether, particularly promestriene.

6. Topical hormonal compositions with a systemic effect according to any one of claims 1 to 5, **characterized in that** the quantity of nomegestrol or of one of its esters or ethers ranges from 0.05 to 5.0 % by weight, of the total composition.

7. Topical hormonal compositions with a systemic effect according to claim 6, **characterized in that** the quantity of nomegestrol or of one of its esters or ethers is of the order of 1.0 % by weight of the total composition.

8. Topical hormonal compositions with a systemic effect according to any one of claims 1 to 7, **characterized in that** the quantity of estradiol, or of one of its esters or ethers, ranges from 0.05 to 1.0% by weight of the total composition.

9. Topical hormonal compositions with a systemic effect according to claim 8, **characterized in that** the quantity of estradiol or of one of its esters or ethers is of the order of 0.15 % by weight of the total composition.

10. Topical hormonal compositions with a systemic effect according to any one of claims 1 to 9, **characterized in that** the solubilizing agent is selected from the group consisting of alcohols, water/alcohol mixtures, propyleneglycol and their mixtures.

11. Topical hormonal compositions with a systemic effect according to any one of claims 1 to 10, **characterized in that** the solubilizing agent is a ethanol / water / propyleneglycol ternary mixture, in which the quantity of ethanol ranges from 30 to 60 % by weight of the total composition, the quantity of water ranges from 20 to 60 % by weight of the total composition and that of propyleneglycol ranges from 2 to 20 % by weight of the total composition.

12. Topical hormonal compositions with a systemic effect according to any one of claims 1 to 11, **characterized in that** the quantity of absorption promoting agent ranges from 2 to 12 % by weight of the total composition.

13. Topical hormonal compositions with a systemic effect according to any one of claims 1 to 12, **characterized in that** the absorption promoting agent is isopropylideneglycerol or 2-*n-*nonyl 1,3-dioxolane.

14. Topical hormonal compositions with a systemic effect according to any one of claims 1 to 13, **characterized in that** the gelling agent is selected from the group constituted by cellulose derivatives and acrylic acid derivatives.

15. Topical hormonal compositions with a systemic effect according to claim 14, **characterized in that** the acrylic derivative is a carbomer with a content of 0.3 to 1 % by weight of the total composition.

16. Topical hormonal compositions with a systemic effect according to any one of claims 1 to 15, **characterized in that** the film-forming agent is selected from the group constituted by silicones, cellulose derivatives, methacrylic derivatives and polyvinylpyrrolidone derivatives.

17. Topical hormonal compositions with a systemic effect according to claim 16, **characterized in that** the silicone is selected from the group constituted by dimethicone, dimethiconol, simethicone and their mixtures.

18. Topical hormonal compositions with a systemic effect according to claim 17, **characterized in that** the silicone is the mixture of dimethiconol in dimethicone .

19. Topical hormonal compositions with a systemic effect according to claim 17 or 18, **characterized in that** the quantity of silicone to be used ranges from 1 to 3 % by weight of the total composition.

20. Topical hormonal compositions according to any one of claims 1 to 19, in the form of a penetrating gel, **characterized in that** it contains, in a hydroalcoholic mixture, 0.4 % nomegestrol acetate, 0.15 % estradiol, 8 % propyleneglycol, 3 % isopropylideneglycerol and 2 % of a mixture of dimethiconol in dimethicone.

21. Topical hormonal compositions according to any one of claims 1 to 19, **characterized in that** it contains, in the form of a penetrating gel, a hydroalcoholic mixture, 0.4 % nomegestrol acetate, 0.15 % estradiol, approximately 8 % propyleneglycol and approximately 3 % isopropylideneglycerol.

## Patentansprüche

1. Topische Hormonzusammensetzung mit systemischer Wirkung zur Hormonbehandlung der Perimenopause und der Menopause sowie zur Behandlung des ovariellen Harmonmangels bei der Frau mit Amenorrhoe auf der Basis eines Progestagens und eines östrogenen Wirkstoffs, **dadurch gekennzeichnet, dass** sie enthält:
- als aktive Wirkstoffe Nomegestrol und/oder einen seiner Ester oder einer seiner Ether als Progestagen und ein Östrogen,
- sowie einen Träger, der die systemische Passage der aktiven Wirkstoffe ermöglicht, gewählt in der Gruppe bestehend aus einem Lösungsvermittler, einem in der Gruppe der Dioxolane gewählten Absorptionsförderer, einem Filmbildner, einem Gelbildner und ihren Mischungen,
in Verbindung oder gemischt mit Exzipienten, die zur Bildung einer gelierten und/oder filmbildenden pharmazeutischen Form geeignet sind.

2. Topische Hormonzusammensetzung mit systemischer Wirkung nach Anspruch 1, **dadurch gekennzeichnet, dass** das Progestagen Nomegetrolacetat ist.

3. Topische Hormonzusammensetzung mit systemischer Wirkung nach Anspruch 1 oder Anspruch 2, **dadurch gekennzeichnet, dass** das Östrogen Östradiol und/oder einer seiner Ester oder einer seiner Ether ist.

4. Topische Hormonzusammensetzung mit systemischer Wirkung nach Anspruch 3, **dadurch gekennzeichnet, dass** das Östrogen ein Fettsäureester von Östradiol, genauer Östradiolvalerat, ist,

5. Topische Hormonzusammensetzung mit systemischer Wirkung nach Anspruch 3, **dadurch gekennzeichnet, dass** das Östrogen ein Östradiolether, insbesondere Promestrien, ist.

6. Topische Hormonzusammensetzung mit systemischer Wirkung nach einem der Ansprüche 1 bis 5, **dadurch gekennzeichnet, dass** die Menge an Nomegestrol oder einem seiner Ester oder Ether von 0,05 bis 5,0 Gewichtsprozent der gesamten Zusammensetzung variiert.

7. Topische Hormonzusammensetzung mit systemischer Wirkung nach Anspruch 6, **dadurch gekennzeichnet, dass** die Menge an Nomegestrol oder einem seiner Ester oder Ether im Bereich von 1,0 Gewichtsprozent der gesamten Zusammensetzung liegt.

8. Topische Hormonzusammensetzung mit systemischer Wirkung nach einem der Ansprüche 1 bis 7, **dadurch gekennzeichnet, dass** die Menge an Östradiol oder einem seiner Ester oder Ether von 0,05 bis 1,0 Gewichtsprozent der gesamten Zusammensetzung variiert.

9. Topische Hormonzusammensetzung mit systemischer Wirkung nach Anspruch 8, **dadurch gekennzeichnet, dass** die Menge an Östradiol oder einem seiner Ester oder Ether im Bereich von 0,15 Gewichtsprozent der gesamten Zusammensetzung liegt.

10. Topische Hormonzusammensetzung mit systemischer Wirkung nach einem der Ansprüche 1 bis 9, **dadurch gekennzeichnet, dass** der Lösungsvermittler in der Gruppe gewählt wird, die aus Alkoholen, hydroalkoholischen Mischungen, Propylenglycol und ihren Mischungen gebildet wird.

11. Topische Hormonzusammensetzung mit systemischer Wirkung nach einem der Ansprüche 1 bis 10, **dadurch gekennzeichnet, dass** der Lösungsvermittler eine ternäre Ethanol/Wasser/Propylenglycol-Mischung ist, in der die Ethanolmenge von 30 bis 60 Gewichtsprozent der gesamten Zusammensetzung variiert, die Wassermenge von 20 bis 60 Gewichtsprozent der gesamten Zusammensetzung variiert und die Propylenglycolmenge von 2 bis 20 Gewichtsprozent der gesamten Zusammensetzung variiert.

12. Topische Hormonzusammensetzung mit systemischer Wirkung nach Anspruch 1 bis 11, **dadurch gekennzeichnet, dass** die Menge an Absorptionsförderer von 2 bis 12 Gewichtsprozent der gesamten Zusammensetzung variiert,

13. Topische Hormonzusammensetzung mit systemischer Wirkung nach Anspruch 1 bis 12, **dadurch gekennzeichnet, dass** der Absorptionsförderer Isopropylidenglycerol oder 2n-Nonyl-1,3-Dioxolan ist.

14. Topische Hormonzusammensetzung mit systemischer Wirkung nach einem der Ansprüche 1 bis 13, **dadurch gekennzeichnet, dass** der Gelbildner in der Gruppe gewählt wird, die aus Cellulosederivaten und Acrylderivaten gebildet wird.

15. Topische Hormonzusammensetzung mit systemischer Wirkung nach Anspruch 14, **dadurch gekennzeichnet, dass** das Acrylderivat ein Carbomer ist, das in einer Konzentration von 0,3 bis 1 Gewichtsprozent der gesamten Zusammensetzung verwendet wird.

16. Topische Hormonzusammensetzung mit systemischer Wirkung nach einem der Ansprüche 1 bis 15, **dadurch gekennzeichnet, dass** der Filmbildner in der Gruppe gewählt wird, die aus Siliconen, Cellulosederivaten, Methacrylderivaten und Polyvinylpyrrolidonderivaten gebildet wird,

17. Topische Hormonzusammensetzung mit systemischer Wirkung nach Anspruch 16, **dadurch gekennzeichnet, dass** das Silicon in der Gruppe gewählt wird, die aus Dimethicon, Dimethiconol, Simethicon und ihren Mischungen gebildet wird.

18. Topische Hormonzusammensetzung mit systemischer Wirkung nach Anspruch 17, **dadurch gekennzeichnet, dass** das Silicon eine Mischung ist, die aus Dimethiconol in Dimethicon gebildet wird.

19. Topische Hormonzusammensetzung mit systemischer Wirkung nach Anspruch 17 oder 18, **dadurch gekennzeichnet, dass** die verwendete Menge an Silicon von 1 bis 3 Gewichtsprozent der gesamten Zusammensetzung variiert.

20. Topische Hormonzusammensetzung nach einem der Ansprüche 1 bis 19 in Form eines penetrierenden Gels, **dadurch gekennzeichnet, dass** sie in einer hydroalkoholischen Mischung enthält: 0,4 % Nomegestrolacetat, 0,15 % Östradiol, 8 % Propylenglycol, 3 % Isoproyplidenglycerol und 2 % einer Mischung, gebildet aus Dimethiconol in Dimethicon.

21. Topische Hormonzusammensetzung nach einem der Ansprüche 1 bis 19, **dadurch gekennzeichnet, dass** sie in Form eines penetrierenden Gels eine hydroalkoholische Mischung, 0,4 % Nomegestrolacetat, 0,15 % Östradiol, etwa 8 % Propylenglycol und etwa 3 % Isoproyplidenglycerol enthält.
